# EUROPÄISCHE PATENTSCHRIFT

(11) **EP 1 095 669 B1**
(45) Veröffentlichungstag und Bekanntmachung des Hinweises auf die Patenterteilung: **23.06.2004**
(21) Anmeldenummer: 00121943.5
(22) Anmeldetag: 09.10.2000
(51) Int. Cl.: A61M 5/32, B23P 19/00

(54) **Vorrichtung zum Aufsetzen von Nadelschutzkappen auf medizinische Spritzen**
Apparatus for placing needle protection sheaths onto medical syringes
Dispositif de placement des capuchons de protection d'aiguille sur des seringues médicales

(30) Priorität: 30.10.1999 DE 19952358
(43) Veröffentlichungstag der Anmeldung: 02.05.2001
(73) Patentinhaber: Arzneimittel GmbH Apotheker Vetter & Co. Ravensburg, D-88212 Ravensburg (DE)
(72) Erfinder: Vetter, Udo J., 88214 Ravensburg (DE); Oliveira, Joachim, 88161 Lindenberg (DE); Sauter, Hubert, 88250 Weingarten (DE)
(74) Vertreter: Dziewior, Joachim, Dipl.-Phys. Dr.

(56) Entgegenhaltungen:
- US-A- 3 187 418
- US-A- 3 623 210
- US-A- 4 070 756
- US-A- 4 271 587

## Beschreibung

Die Erfindung betrifft eine Vorrichtung zum Aufsetzen von Nadelschutzkappen auf medizinische Spritzen, insbesondere auf Spritzen mit eingeklebter Kanüle, mit einem Halteelement für eine oder mehrere Spritzen sowie einem Zuführelement für die Nadelschutzkappen und einer Verschlußeinrichtung (10), die sich zum Aufsetzen der Nadelschutzkappe öffnen läßt, wobei das Halteelement und das Zuführelement zum Aufsetzen der Nadelschutzkappen gegeneinander verstellbar sind.

Eine Solche Vorrichtung ist zum Beispiel aus US-A-4070756 bekannt geworden.

Nadelschutzkappen bestehen üblicherweise aus gummielastischem Material und dienen dazu, insbesondere bei Spritzen mit eingeklebter Kanüle diese vor Beschädigungen zu schützen. Dazu besitzt die Nadelschutzkappe in der Regel eine langgestreckte Form, mit einem der Länge der Kanüle angepaßten, zur Spitze hin leicht konischen Innenraum. Auch die äußere Mantelfläche der Nadelschutzkappe kann konische Gestalt aufweisen, zumindest besitzt die Nadelschutzkappe an ihrem offenen Ende meist einen radial nach außen vorstehenden Ringbund.

Dies hat zur Folge, daß die meist durch Teflonschläuche oder in ähnlicher Weise zur Aufsetzstation zugeführten Nadelschutzkappen schräg auf den Konus der Nadelschutzkappen aufgesetzt werden, da sie während des Transports häufig einseitig an der Wand der Fördermittel anliegen, im Ergebnis also bereits schräg zugeführt werden. Ursache hierfür sind insbesondere statische Aufladungen der Nadelschutzkappe, die sich durch die Bewegungen in den Fördermitteln ergeben und aufsummieren.

Wird eine derartig schräg auf dem Konus der Spritze aufgesetzte Nadelschutzkappe vollständig aufgesteckt, so besteht die Gefahr, daß die Kanüle seitlich in die Nadelschutzkappe einsticht und diese gegebenenfalls durchsticht. Spritzen mit durchstochenen Nadelschutzkappen dürfen jedoch aus Sicherheitsgründen nicht weiterverarbeitet werden, da zum einen die Sterilität nicht mehr gegeben ist, andererseits die Gefahr von Verletzungen des Anwenders beim Abziehen der Nadelschutzkappe vor der Injektion besteht. Darüberhinaus besteht die Gefahr, daß durch die Kanüle Gummiteile aus der Nadelschutzkappe ausgestanzt werden, die später entweder die Kanüle verstopfen oder sogar mit der Injektion in den Körper des Patienten gelangen können.

Der Erfindung liegt die Aufgabe zugrunde, eine Vorrichtung der eingangs genannten Art zu schaffen, die es ermöglicht, den Anteil an schräg aufgesetzten Nadelschutzkappen auf ein Minimum zu reduzieren, wobei gleichzeitig jedoch keine Einschränkung hinsichtlich des zeitlichen Durchsatzes beim Aufsetzen der Nadelschutzkappen erfolgen soll.

Diese Aufgabe wird nach der Erfindung dadurch gelöst, daß das Zuführelement für jede Nadelschutzkappe eine Führungshülse aufweist, deren Durchmesser größer als der maximale Außendurchmesser der Nadelschutzkappe ist, wobei in die Führungshülse wenigstens eine Druckluftzuführung mündet, über die Luft in den zwischen der Wand der Führungshülse und der Mantelfläche der Nadelschutzkapppe gebildeten Ringraum eingeblasen werden kann, und daß zwischen dem Halteelement und der nach unten offenen Führungshülse die Verschlußeinrichtung angeordnet ist.

Der durch die Erfindung erreichte Vorteil besteht im wesentlichen darin, daß die Nadelschutzkappen zunächst in der Führungshülse durch den eintretenden Luftstrom in eine Rotationsbewegung versetzt werden, so daß sie sich trotz eventueller statischer Aufladungen vollständig von der Seitenwand lösen. Dieses Anblasen mit Preßluft hat darüberhinaus zur Folge, daß die Nadelschutzkappen auf einem sich ausbildenden Luftpolster schweben. Im übrigen ergibt sich aus der Rotation der Nadelschutzkappen über den aufgenommenen Drehimpuls eine Stabilisierung in ihrer aktuellen Lage, die sie beim anschließenden Aufsetzen auf die Spritze weitestgehend beibehalten. Im übrigen erfolgt durch die induzierte Rotation der Nadelschutzkappe eine exakte Zentrierung bezüglich der Kanüle bzw. dem Konus der Spritze.

Nach dem Öffnen der Verschlußeinrichtung fällt somit die Nadelschutzkappe im freien Fall exakt auf den Konus der Spritze.

In bevorzugter Ausführungsform der Erfindung ist vorgesehen, daß jede Druckluftzuführung annähernd tangential in die Führungshülse einmündet. Auf diese Weise wird in dem Ringraum eine maximale Drehströmung erreicht.

Weiterhin hat es sich im Rahmen der Erfindung als vorteilhaft herausgestellt, wenn zwei (oder mehrere) Druckluftzuführungen vorgesehen sind, deren Mündungen etwa diametral zueinander angeordnet sind.

Nach einer ersten vorteilhaften Weiterbildung kann die Verschlußeinrichtung an jeder der Führungshülsen von einer irisartigen Blende gebildet sein, die zwischen einer ganz oder zumindest nahezu geschlossenen und einer geöffneten, den Durchtritt der Nadelschutzkappe ermöglichenden Stellung verstellbar ist.

Es besteht jedoch ebenso die Möglichkeit, daß die Verschlußeinrichtung von einer oder zwei sich gegenläufig bewegenden Schiebeleisten gebildet ist, die in horizontaler Richtung verstellbar sind.

Schließlich besteht auch die Möglichkeit, daß die Verschlußeinrichtung von einer oder zwei in gegenläufigem Drehsinn beweglichen Schwenkleisten gebildet ist, die um horizontale Gelenkwellen drehbar sind.

In folgenden wird die Erfindung an in der Zeichnung dargestellten Ausführungsbeispielen näher erläutert; es zeigen:
- Fig. 1: eine schematische Darstellung der Vorrichtung in Seitenansicht,
- Fig. 2: einen Schnitt durch den Gegenstand nach Fig. 1 längs der Linie A-A,
- Fig. 3: eine Draufsicht auf eine erste Ausführungsform der Verschlußeinrichtung für eine Führungshülse von unten,
- Fig. 4: und 5 weitere Ausgestaltungen der Verschlußeinrichtung in der Fig. 3 entsprechender Darstellung,
- Fig. 6: eine weitere Ausgestaltung der Verschlußeinrichtung in der Fig. 1 entsprechender Seitenansicht.

Die in der Zeichnung nur schematisch dargestellte Vorrichtung zum Aufsetzen von Nadelschutzkappen 1 auf medizinische Spritzen 2 besteht im wesentlichen aus einem Halteelement 3 für eine oder mehrere Spritzen 2 sowie aus einem Zuführelement 4 für die Nadelschutzkappen 1.

Im Rahmen der Fertigung der insbesondere mit eingeklebter Kanüle 5 ausgebildeten Spritzen 2 werden die Nadelschutzkappen 1 in in der Zeichnung nicht näher dargestellter Weise über Rundförderer, Längsförderer und schließlich durch Teflonschläuche hindurch der Vorrichtung zugeführt. Bei diesem Transport erfahren die Nadelschutzkappen 1 häufig statische Aufladungen, die sich aus der Bewegung und insbesondere der Reibung der Nadelschutzkappe 1 ergeben und aufsummieren. In Folge dieser statischen Aufladung bleiben die Nadelschutzkappen 1 schließlich an der Seitenwand der Aufsetzvorrichtung in schräger Lage hängen, so daß sie schließlich in dieser Schräglage auf den Konus 6 der Spritze 2 aufgesetzt werden.

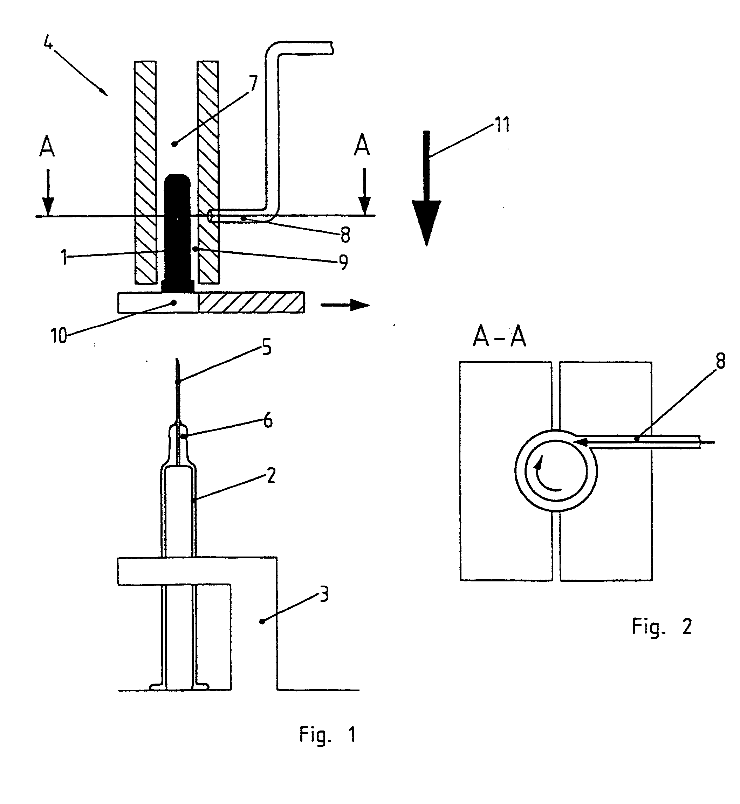

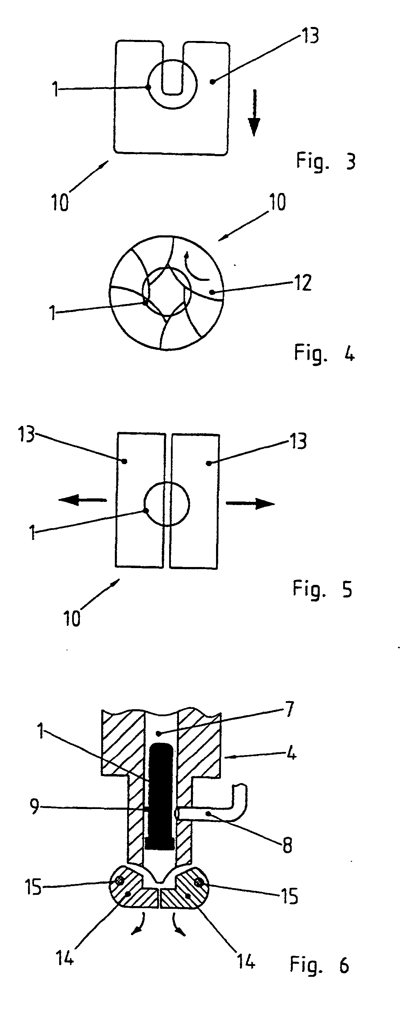

Um dies zu vermeiden, weist das Zuführelement 4 nach der Erfindung für jede Nadelschutzkappe 1 eine Führungshülse 7 auf, deren Durchmesser größer ist als der maximale Außendurchmesser der Nadelschutzkappe 1. In die Führungshülse 7 mündet, wie in den Fig. 1 und 2 dargestellt ist, wenigstens eine Druckluftzuführung 8, über die Luft in den zwischen der Wand der Führungshülse 7 und der Mantelfläche der Nadelschutzkappe 1 gebildeten Ringraum 9 eingeblasen werden kann. Hierdurch wird die Nadelschutzkappe 1 zunächst von der Wand gelöst und darüberhinaus in Rotation versetzt, wodurch sich eine sehr stabile Ausrichtung der Nadelschutzkappe 1 ergibt. Darüberhinaus erfolgt eine exakte Zentrierung der Nadelschutzkappe 1 zu der im Halteelement 3 angeordneten Spritze 2, so daß die Nadelschutzkappe 1 nach dem Öffnen der unterhalb der nach unten offenen Führungshülse 7 angeordneten Verschlußeinrichtung 10 die Nadelschutzkappe 1 axial ausgerichtet auf den Konus 6 der Spritze 2 fällt.

Um danach die Nadelschutzkappe 1 vollständig frei zu geben, sind das Halteelement 3 und das Zuführelement 4 in vertikaler Richtung gegeneinander verstellbar, wie dies durch den Pfeil 11 in Fig. 1 angedeutet ist.

Im Anschluß hieran können die Spritzen 2 in einer nicht dargestellten nachfolgenden Station durch einen Nadelschutzkappen-Rüttler in geringe translatorische Schwingungen versetzt werden, wodurch sich eine ergänzende Ausrichtung der Nadelschutzkappe 1 auf dem Konus 6 ergibt. In ebenfalls nicht näher dargestellter Weise kann schließlich in einer Nachdrückstation die Nadelschutzkappe 1 endgültig und vollständig auf den Konus 6 der Spritze 2 aufgeschoben werden.

Wie sich aus der Fig. 2 ersehen läßt, mündet die Druckluftzuführung 8 annähernd tangential in die Führungshülse 7, so daß eine weitgehend wirbelfreie Rotationsbewegung im Ringraum 9 erreicht wird. Hierfür ist es im übrigen in nicht näher dargestellter Weise vorteilhaft, wenn zwei Druckluftzuführungen 8 vorgesehen sind, deren Mündungen etwa diametral zueinander angeordnet sind.

In Fig. 4 ist die Verschlußeinrichtung 10 an jeder der Führungshülsen von einer irisartigen Blende 12 gebildet. Diese Blende 12 kann zwischen einer ganz oder zumindest nahezu geschlossenen Stellung in eine geöffnete Stellung gebracht werden, die dann zum Aufsetzen der Nadelschutzkappe 1 deren Durchtritt ermöglicht.

Gemäß den Fig. 3 und 5 kann die Verschlußeinrichtung 10 jedoch auch von einer oder zwei sich gegenläufig bewegenden Schiebeleisten 13 gebildet sein, die in horitzontaler Richtung verstellbar sind.

Schließlich besteht auch die in Fig. 6 dargestellte Möglichkeit, daß die Verschlußeinrichtung 10 von zwei in gegenläufigem Drehsinn - gegebenenfalls auch nur einer - beweglichen Schwenkleisten 14 gebildet ist, die um horizontale Gelenkwellen 15 drehbar sind.

## Patentansprüche

1. Vorrichtung zum Aufsetzen von Nadelschutzkappen (1) auf medizinische Spritzen (2), insbesondere auf Spritzen (2) mit eingeklebter Kanüle (5), mit einem Halteelement (3) für eine oder mehrere Spritzen (2) sowie einem Zuführelement (4) für die Nadelschutzkappen (1) und einer Verschlußeinrichtung (10) die sich zum Aufsetzen der Nadelschutzkappe (1) öffnen läßt, wobei das Halteelement (3) und das Zuführelement (4) zum Aufsetzen der Nadelschutzkappen (1) gegeneinander verstellbar sind, **dadurch gekennzeichnet, daß** das Zuführelement (4) für jede Nadelschutzkappe (1) eine Führungshülse (7) aufweist, deren Durchmesser größer als der maximale Außendurchmesser der Nadelschutzkappe (1) ist, wobei in die Führungshülse (7) wenigstens eine Druckluftzuführung (8) mündet, über die Luft in den zwischen der Wand der Führungshülse (7) und der Mantelfläche der Nadelschutzkappe (1) gebildeten Ringraum (9) eingeblasen werden kann, und daß die Verschlußeinrichtung (10) zwischen dem Halteelement (3) und der nach unten offenen Führungshülse (7) angeordnet ist.

2. Vorrichtung nach Anspruch 1, **dadurch gekennzeichnet, daß** jede Druckluftzuführung (8) annähernd tangential in die Führungshülse (7) einmündet.

3. Vorrichtung nach Anspruch 1 oder 2, **dadurch gekennzeichnet, daß** zwei Druckluftzuführungen (8) vorgesehen sind, deren Mündungen etwa diametral zueinander angeordnet sind.

4. Vorrichtung nach einem der Ansprüche 1 bis 3, **dadurch gekennzeichnet, daß** die Verschlußeinrichtung (10) an jeder der Führungshülsen (7) von einer irisartigen Blende (12) gebildet ist, die zwischen einer ganz oder zumindest nahezu geschlossenen und einer geöffneten, den Durchtritt der Nadelschutzkappe (1) ermöglichenden Stellung verstellbar ist.

5. Vorrichtung nach einem der Ansprüche 1 bis 3, **dadurch gekennzeichnet, daß** die Verschlußeinrichtung (10) von einer oder zwei sich gegenläufig bewegenden Schiebeleisten (13) gebildet ist, die in horizontaler Richtung verstellbar sind.

6. Vorrichtung nach einem der Ansprüche 1 bis 3, **dadurch gekennzeichnet, daß** die Verschlußeinrichtung (10) von einer oder zwei in gegenläufigem Drehsinn beweglichen Schwenkleisten (14) gebildet ist, die um horizontale Gelenkwellen (15) drehbar sind.

## Claims

1. An apparatus for fitting needle protection caps (1) onto medical syringes (2), in particular onto syringes (2) with a needle (5) which is glued in place, comprising a holding element (3) for one or more syringes (2) and a feed element (4) for the needle protection caps (1), and a closure device (10) which can be opened for fitting the needle protection cap (1), wherein the holding element (3) and the feed element (4) are displaceable relative to each other for fitting the needle protection caps (1), **characterised in that** the feed element (4) has for each needle protection cap (1) a guide sleeve (7) whose diameter is larger than the maximum outside diameter of the needle protection cap (1), wherein opening into the guide sleeve (7) is at least one compressed air feed (8), by way of which air can be injected into the annular space (9) formed between the wall of the guide sleeve (7) and the peripheral surface of the needle protection cap (1), and that the closure device (10) is arranged between the holding element (3) and the downwardly open guide sleeve (7).

2. Apparatus according to claim 1 **characterised in that** each compressed air feed (8) opens approximately tangentially into the guide sleeve (7).

3. Apparatus according to claim 1 or claim 2 **characterised in that** two compressed air feeds (8) are provided, the mouth openings of which are arranged in approximately diametral relationship.

4. Apparatus according to one of claims 1 to 3 **characterised in that** the closure device (10) can be formed on each of the guide sleeves (7) by an iris-like aperture member (12) which is displaceable between an entirely or at least almost closed position and an opened position for permitting the needle protection cap (1) to pass therethrough.

5. Apparatus according to one of claims 1 to 3 **characterised in that** the closure device (10) is formed by one or two oppositely movable slide plates (13) which are displaceable in the horizontal direction.

6. Apparatus according to one of claims 1 to 3 **characterised in that** the closure device (10) is formed by one or two pivotal plates (14) which are movable in opposite directions of rotation and which are rotatable about horizontal pivot shafts (15).

## Revendications

1. Dispositif de placement de capuchon de protection d'aiguille (1) sur des seringues médicales (2), notamment sur des seringues équipées d'aiguilles collées (5), qui comprend un élément de maintien (3) pour une ou plusieurs seringues (2), ainsi qu'un élément d'amenée (4) des capuchons de protection (1) et un dispositif de fermeture (10) qui peut s'ouvrir pour placer le capuchon de protection (1), l'élément de maintien (3) et l'élément d'amenée (4) pouvant être déplacés l'un par rapport à l'autre pour permettre le placement des capuchons d'aiguille (1), **caractérisé en ce que**
- l'élément d'amenée (4) présente, pour chaque capuchon de protection d'aiguille (1), une douille de guidage (7) dont le diamètre est supérieur au diamètre externe maximal du capuchon de protection d'aiguille (1)
- dans la douille de guidage (7), débouche au moins une amenée d'air comprimé (8) permettant de souffler l'air dans l'espace annulaire (9) formé entre la paroi de la douille de guidage (7) et la surface externe du capuchon de protection d'aiguille (1)
- le dispositif de fermeture (10) est disposé entre l'élément de maintien (3) et la douille de guidage (7) ouverte vers le bas.

2. Dispositif selon la revendication 1, **caractérisé en ce que** chaque amenée d'air comprimé (8) débouche à peu près tangentiellement dans la douille de guidage (7).

3. Dispositif selon la revendication 1 ou 2, **caractérisé en ce que** il est prévu deux amenées d'air comprimé (8) dont les embouchures sont à peu prés diamétralement opposées.

4. Dispositif selon une des revendications 1 à 3, **caractérisé en ce que** le dispositif de fermeture (10) sur chacune des douilles de guidage (7) est constitué par un diaphragme (12) du genre iris, qui peut se déplacer entre une position totalement ou à peu près fermée, à une position ouverte permettant le passage du capuchon de protection d'aiguille.

5. Dispositif selon une des revendications 1 à 3, **caractérisé en ce que** le dispositif de fermeture (10) est constitué d'une ou de deux barrettes coulissantes (13) se déplaçant en sens opposés, selon une direction horizontale.

6. Dispositif selon une des revendications 1 à 3, **caractérisé en ce que** le dispositif de fermeture (10) est constitué par une ou deux barrettes basculantes (14) tournant dans des sens opposés autour d'axes d'articulation horizontaux (15).
